# EUROPÄISCHE PATENTSCHRIFT

(11) **EP 1 452 144 B1**
(45) Veröffentlichungstag und Bekanntmachung des Hinweises auf die Patenterteilung: **21.11.2007**
(21) Anmeldenummer: 04000484.8
(22) Anmeldetag: 13.01.2004
(51) Int. Cl.: A61B 17/58, A61B 17/78

(54) **Verriegelungsnagel, insbesondere für Frakturen der proximalen Femur**
Locking nail particulary for fractures of the proximal end of the femur
Clou de verrouillage, en particulier pour les fractures de l'extrémité proximale du fémur

(30) Priorität: 07.02.2003 DE 20301902 U
(43) Veröffentlichungstag der Anmeldung: 01.09.2004
(60) Teilanmeldung: 07017392.7
(73) Patentinhaber: Stryker Trauma GmbH, 24232 Schönkirchen (DE)
(72) Erfinder: Speitling, Andreas, 24149 Kiel (DE); Dorawa, Klaus, 24232 Schönkirchen (DE); Von Oldenburg, Geert, 24226 Heikendorf (DE)
(74) Vertreter: Maiwald Patentanwalts GmbH

(56) Entgegenhaltungen:
- DE-C1- 4 341 677
- GB-A- 2 209 947
- US-B1- 6 224 601

## Beschreibung

Die Erfindung bezieht sich auf einen Verriegelungsnagel, insbesondere für Frakturen der proximalen Femur nach dem Oberbegriff des Anspruchs 1.

Es ist bekannt, zur Versorgung von Trochanterfrakturen und Frakturen des Schenkelhalses oder des Femurkopfes einen Verriegelungsnagel vorzusehen, der von proximal in den Femur eingetrieben wird und der in einer Schrägdurchbohrung einen Schenkelhalsstift führt, der über den Schenkelhals des Femurs in den Femurkopf eingeführt wird. Es ist bekannt, den Schenkelhalsstift mit einem Gewinde zu versehen, damit er in den Femurkopf eingeschraubt werden kann (Schenkelhalsschraube); es ist jedoch auch bekannt, den Schenkelhalsstift als Klinge auszuführen. Ferner ist bekannt, im proximalen Teil des Verriegelungsnagels einen Verriegelungsstift vorzusehen, der mit dem Schenkelhalsstift so zusammenwirkt, dass dieser sich zwar axial in der Schrägdurchbohrung bewegen, sich jedoch nicht drehen kann.

Die Gewichtskraft eines mit einem derartigen Implantat versorgten Patienten wird im wesentlichen von dem Schenkelhalsstift in den Verriegelungsnagel eingeleitet. Es kommt zu einer zusammengesetzten Spannung im belasteten Nagelquerschnitt, die sich aus Biege- und Zugspannungen zusammensetzt. Bei Überlast ist eine Rissbildung zu befürchten, und zwar an der Stelle, an der die höchste Zugspannung auftritt. Die höchste Spannungskonzentration entsteht an den scharfen seitlichen Kanten auf gegenüberliegenden Seiten des Schenkelhalsstiftes, wenn er sich in der Schrägdurchbohrung befindet. Die sogenannte Zeitfestigkeit des Implantats ist mithin abhängig von dem kritischen Bereich mit der scharfkantigen Geometrie der Kanten. Die größten Zugspannungen treten naturgemäß am Eintrittsende der Bohrung auf.

Aus US 6,224,601 ist ein Osteosynthese-Hilfsmittel mit einem Verriegelungsnagel bekannt, welcher in einen medullären Bereich eines Femurs eingeführt werden kann, und welcher einen Distalbereich mit zumindest einem länglichen Loch und einen Proximalbereich aufweist, welcher Proximalbereich ein schräges Loch aufweist.

Der Erfindung liegt die Aufgabe zugrunde, einen Verriegelungsnagel der genannten Art dahingehend zu verbessern, daß seine Zeitfestigkeit erhöht wird.

Diese Aufgabe wird durch die Merkmale des Patentanspruchs 1 gelöst.

Bei dem erfindungsgemäßen Verriegelungsnagel sind die auf gegenüberliegenden Seiten des Stiftes (wenn er sich in der Bohrung befindet) liegenden Kanten am Eintrittsende und/oder Austrittsende der Bohrung abgeflacht oder angefast. Sie gehen gerundet und stetig in die benachbarten proximalen und distalen Kantenabschnitte über. Vorzugsweise sind die durch die abgeflachten Kanten gebildeten Flächenabschnitte im distalen und proximalen Endbereich in Seitenansicht im wesentlichen konkav ausgebildet und im Querschnitt annähernd gerade.

Bezüglich der einzelnen Kantenabschnitte am Eintrittsende der Bohrung werden die zum proximalen und zum distalen Ende des Nagels hin gerichteten Kantenabschnitte von solchen unterschieden, die zu den Seiten des Nagels hin liegen. Die letzteren sind im Hinblick auf die Ausbildung von Flächenabschnitten in erster Linie gemeint. Denn bei den bekannten Nägeln stellen diese Kantenabschnitte für die Belastung die am meisten kritischen Abschnitte dar, weil sie Bereich reduzierten Querschnittes liegen. Bei der Erfindung wird im Bereich dieser Kantenabschnitte durch Anfasung etwas Material fortgenommen in der Weise, daß eine Kerbwirkung und damit die Gefahr der Bildung von Spannungsspitzen in diesem Bereich vermieden wird. Trotz der Materialentfernung wird die Belastbarkeit erhöht. Darüber hinaus wird im wesentlichen die gesamte Auflagefläche für den Stift in der Bohrung, insbesondere des Schenkelhalsstiftes in der Schrägdurchbohrung wie sie durch die Geometrie des Nagels und der Bohrung vorgegeben ist, beibehalten.

Es ist zwar denkbar, ausschließlich in den angesprochenen Kantenbereichen eine Materialentfernung durch eine Abflachung oder Abschrägung vorzusehen. Es ist jedoch vorzuziehen, wenn nach einer Ausgestaltung der Erfindung am Eintritts- oder Austrittsende der Bohrung zur Bildung der abgeflachten Kantenabschnitte eine Kerbe geformt ist, welche nahezu die gesamte umlaufende Kante zumindest des Eintrittsendes anschrägt. Auch diese kann so geometrisch gestaltet sein, daß die Gleitfläche des Stifts im wesentlich auf ganzer Länge erhalten bleibt.

Vorzugsweise bildet die äußere gerundete umlaufende Kante der Kerbe eine im wesentlichen rechteckige oder quadratische Kontur mit abgerundeten Ecken.

Die relativ schmalen, länglichen Flächenabschnitte an den gegenüberliegenden seitlichen Kantenabschnitten gehen vorzugsweise in geschrägte oder gefaste Flächenabschnitte zur proximalen und distalen Seite des Eintritts- oder Austrittsende hin über, die - seitlich gesehen - einen konkaven Verlauf aufweisen und gerundet in die gerundete äußere Kante übergehen.

Die Erfindung ist insbesondere für Verriegelungsnägel geeignet, die in einer proximalen Schrägbohrung einen Schenkelhalsstift aufnehmen. Hierbei ist auch die erfindungsgemäße Entlastung des Eintrittsendes der Schrägbohrung ausreichend. Sie ist jedoch auch für Bohrungen des Verriegelungsnagels geeignet, die senkrecht zur Nagelachse liegen und distal angeordnet sind.

Beim erfindungsgemäßen Nagel wird die Zeitfestigkeit des Implantats erhöht, indem die Außengeometrie der Kanten der Bohrung an den Enden so verändert werden, daß die durch die Belastung im klinischen Einsatz hervorrufenden Spannungen im Material umgeleitet werden, um besonders kritische Bereiche, wie sie mit scharfkantigen Geometrien entstehen, zu entlasten. Die scharfen Kanten werden entfernt und die Spannung wird über eine größere Fläche verteilt. Dieser Vorteil wird erzielt, ohne daß der Gleitmechanismus für den Stift beeinträchtigt ist.

Wie schon erläutert, verlaufen die Spannungslinien bei einer Durchbohrung in einem Nagel entlang des schwächsten Querschnitts. Dadurch, daß bei der Erfindung eine Abflachung in den seitlichen Kantenabschnitten des Eintrittsendes der Schrägbohrung erfolgt, werden die Spannungslinien im Bereich höherer Festigkeit um den Querschnitt umgeleitet. Hierbei kann auch vorgesehen werden, die umlaufende Kante am Eintrittsende der Bohrung über ihren gesamten Verlauf durch Materialentfernung anzufasen oder anzuschrägen.

Die Erfindung wird nachfolgend anhand eines in Zeichnungen dargestellten Ausführungsbeispiels näher erläutert.
- Fig. 1: zeigt die Seitenansicht eines Verriegelungsnagels nach der Erfindung,
- Fig. 2: zeigt einen Schnitt durch den Nagel nach Fig. 1 entlang der Linie 2-2,
- Fig. 3: zeigt vergrößert die Draufsicht auf einen Teil des Nagels nach Fig. 1,
- Fig. 4: zeigt eine ähnliche Draufsicht wie Fig. 3, jedoch aus einer um 90° versetzten Perspektive,
- Fig. 5: zeigt die Seitenansicht des Nagelabschnitts nach Fig. 3,
- Fig. 6: zeigt eine Draufsicht auf einen proximalen Abschnitt eines Knochennagels ähnlich den proximalen Abschnitten nach den Fign. 3 bis 5 mit einer Schrägdurchbohrung nach dem Stand der Technik,
- Fig. 7: zeigt die gleiche Ansicht nach einer Anfasung der umlaufenden Kante des Eintrittsendes der gezeigten Schrägbohrung,
- Fig. 8: zeigt eine Seitenansicht des Nagelabschnitts nach Fig. 7.

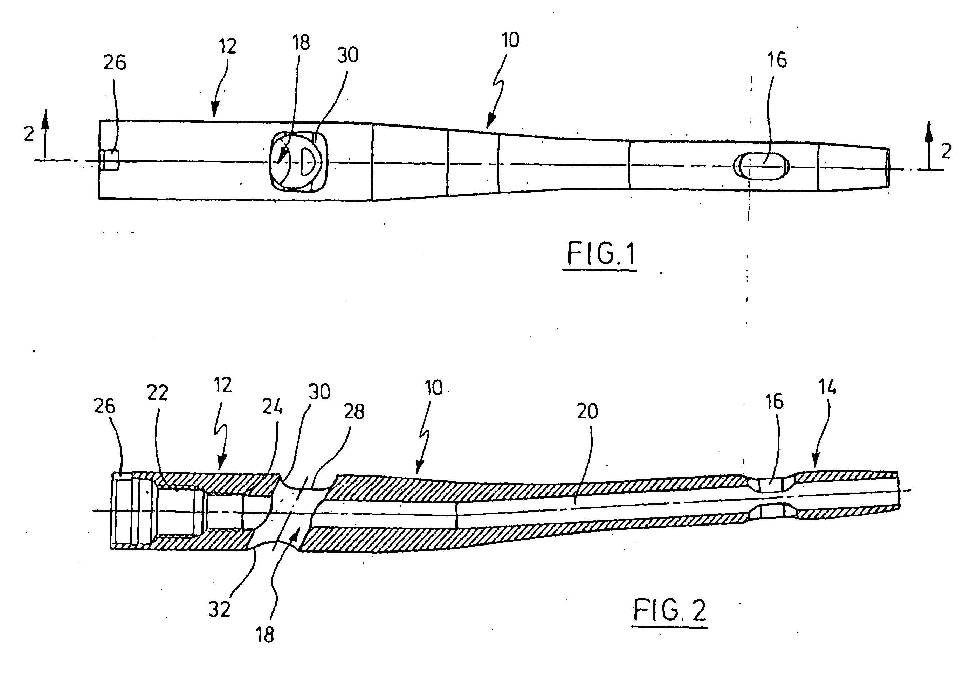

Einer in den Fign. 1 und 2 dargestellter Verriegelungsnagel 10 weist einen proximalen Abschnitt 12 und einen distalen Abschnitt 14 auf. Letzterer enthält eine längliche Querbohrung 16 zur Aufnahme einer nicht dargestellten Verriegelungsschraube. Der proximale Abschnitt weist eine Schrägdurchbohrung 18 auf zur Aufnahme eines nicht gezeigten Schenkelhalsstiftes. Vorstehend und nachstehend ist stets von einem Schenkelhalsstift die Rede, wobei dieser allgemein alle üblichen Schenkelhalsschrauben und -stifte erfassen soll, welche bisher bekannt geworden sind.

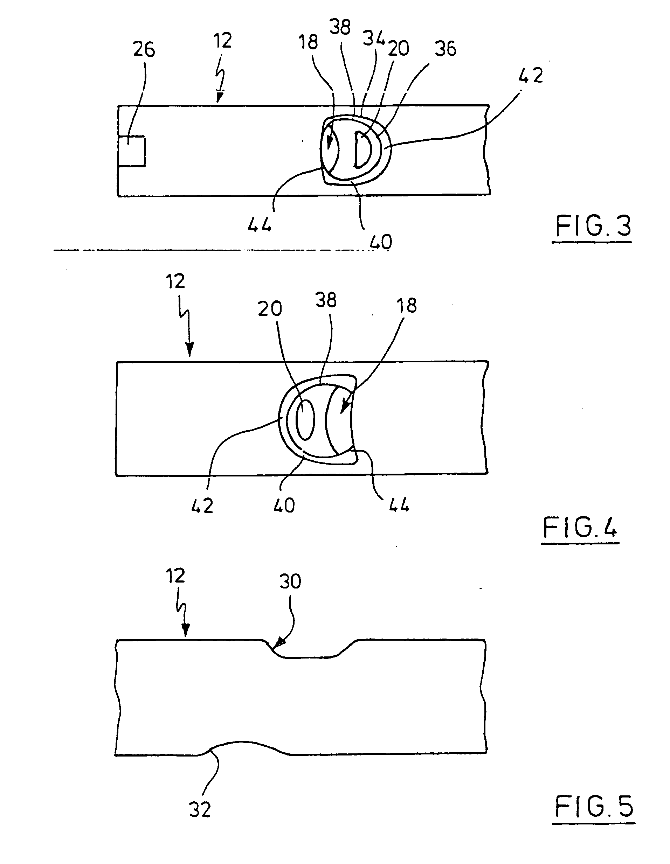

Der Nagel 10 ist komplett mit einer axialen Durchbohrung 20 geformt, und am proximalen Ende sind zwei im Durchmesser unterschiedliche Gewindeabschnitte 22, 24 vorgesehen, wobei der innen liegende zur Aufnahme eines nicht gezeigten Verriegelungsstifts für den Schenkelhalsstift dient und das Gewinde 22 zur Aufnahme eines nicht gezeigten Einschlag- und Zielinstruments zur Anbringung des Nagels 10 über den proximalen Femur. Eine radiale Ausnehmung 26 am proximalen Ende dient zur Orientierung des Einschlag- und Zielinstruments am Nagel 10 in Drehrichtung. Die erwähnten konstruktiven Merkmale sind Stand der Technik. Die nachfolgende Beschreibung richtet sich auf die Schrägbohrung 18. Diese hat ein Eintrittsende 30 und ein-Austrittsende 32 für-den nicht gezeigten Schenkelhalsstift. Das Eintrittsende ist vom Kopf des Femurs abgewandt, wenn der Nagel proximal in den Femur eingetrieben ist. Die nachfolgenden Erörterungen beziehen sich ausschließlich auf das Eintrittsende, das in Fig. 1 zu erkennen, jedoch in den Fign. 3 und 4 deutlicher herausgestellt ist.

Wie aus den Figuren hervorgeht, ist im Bereich des Eintrittsendes 30 in Draufsicht eine Kerbe eingeformt, deren äußere gerundete Kante 34 annähernd quadratische Kontur hat mit abgerundeten Ecken. Die Kante 34 liegt naturgemäß in der Außenkontur des proximalen Nagelabschnitts 12 und außerhalb der Kante, welche durch die Bohrung 18 gebildet wird. Die radial innerhalb der äußeren Kante 34 liegende innere Kante der Kerbe, welche durch die Bohrung 18 gebildet ist, ist mit 36 bezeichnet. Zwischen der äußeren Kante 34 und der inneren Kante 36 sind zum Teil abgeschrägte oder gefaste Flächenabschnitte ausgebildet. Erste seitliche Flächenabschnitte 38, 40 liegen auf gegenüberliegenden Seiten des nicht gezeigten Schenkelhalsstiftes und erstrecken sich in Draufsicht annähernd parallel zur Außenseite des proximalen Abschnitts 12. Die Flächenabschnitte 38, 40 liegen zumindest im mittleren Bereich annähernd in einer gemeinsamen Ebene, wie sich aus Fig. 2 ergibt, die annähernd parallel zur Längsachse des proximalen Abschnitts 12 verläuft. Nach proximal und distal hin gehen die Flächenabschnitte 38, 40 stetig in Flächenabschnitte 42, 44 über. Der Flächenabschnitt 42 liegt distal und der Flächenabschnitt 44 proximal. Wie aus Fig. 2 zu erkennen, ist der Konturverlauf der Flächenabschnitte 38, 40 im Längsschnitt schwach konkav, insbesondere zu den Enden hin und die Flächenabschnitte 38, 40 gehen stetig in einen Flächenabschnitt über, der proximal durch die Bohrung 18 gebildet ist. Der äußere proximale Kantenabschnitt entspricht im wesentlichen dem Verlauf des Kantenabschnitts, der ohnehin durch die Bohrung 18 gebildet ist.

Die beschriebene Formung oder Auskerbung im Eintrittsbereich für den Schenkelhalsstift in die Schrägbohrung 18 ist fertigungstechnisch relativ einfach herstellbar und sichert eine deutliche Reduzierung der Spannungsspitzen in den Endbereichen der Bohrung 18 bei der Belastung des Schenkelhalsstiftes in der Bohrung 18 durch die Gewichtskraft des Patienten, insbesondere am Eintrittsende, ohne daß die Gleitfläche des Schenkelhalsstiftes, d.h. die Auflagefläche für den Schenkelhalsstift in der Schrägbohrung 18, merklich verringert ist. Die Materialwegnahme am Eintrittsende 30 läßt sich anhand von Fig. 5 leicht erkennen. Die Durchdringungslinie der Schrägbohrung 18 in der Außenkontur des proximalen Nagelabschnitts 12 ist durch die Materialwegnahme bzw. Anfasung der Kantenbereiche des Eintrittsendes 30 abgewandelt. Man erkennt, daß relativ wenig Material entfernt wurde, so daß die Materialschwächung hierdurch vernachlässigbar ist und durch die Vorteile der günstigen Krafteinleitung vom Schenkelhalsstift in den Nagel weit überwogen wird. Entscheidend für die Reduzierung der Spannungsspitzen sind die Flächenabschnitte 38,40.

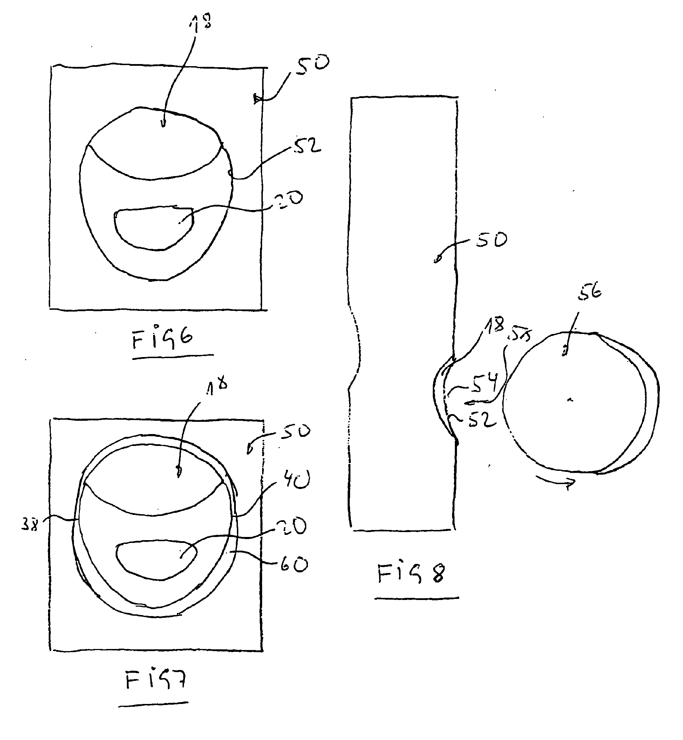

In Fign. 6 bis 8 ist ein proximaler Nagelabschnitt 50 dargestellt, etwa eines Femurnagels gemäß den Fign. 1 und 2. In den Fign. 6 und 7 ist das jeweils obere Ende zum proximalen Ende des Nagels hin gezeigt, während das untere Ende zum distalen Ende hin zeigt. Die Schrägbohrung ist ebenfalls mit 18 angegeben. Auch die axiale Durchbohrung ist mit dem gleichen Bezugszeichen 20 wie in den Fign. 2 bis 4 angegeben. Die Blickrichtung der Seitenansichten nach den Fign. 6 und 7 ist senkrecht zur Nagelachse. Fig. 6 zeigt den Zustand des Nagelabschnitts 50 nach dem Herstellen der Schrägdurchbohrung und der axialen Durchbohrung 20. Fig. 7 zeigt die Verformung der umlaufenden Kante 52 des Eintrittsendes für einen nicht gezeigten Schenkelhalsstift, der in die Schrägbohrung 18 eingeführt wird nach dem Anfasen oder der Abflachung mit einem Fräswerkzeug, wie dies anhand von Fig. 8 noch beschrieben werden soll.

Fig. 8 zeigt die Seitenansicht des Nagelabschnitts 50 annähernd senkrecht zur Schrägdurchbohrung 18, wobei jedoch der Nagelabschnitt 50 um etwa 5 DEG in Uhrzeigerrichtung verdreht worden ist. Dadurch wird am Eintrittsende 54 der Schrägbohrung 18 der Verlauf der Kante 52 deutlicher erkennbar. In Fig. 8 ist ferner ein kreisförmiges Fräswerkzeug 56 zu erkennen, das um eine Achse gedreht wird, die senkrecht auf der Achse des Nagelabschnitts 50 steht. Die axiale Erstreckung des Fräswerkzeugs 56 ist grösser als der Durchmesser der Schrägbohrung 18. Der Durchmesser des kreisförmigen Fräswerkzeugs 56 ist ebenfalls grösser als der Durchmesser der Schrägbohrung 18. Bei der Bearbeitung wird das Fräswerkzeug in Richtung des Pfeils 58 auf die Kante 52 zu bewegt. Das Fräswerkzeug 56 taucht dabei annähernd mittig in das Eintrittsende 54 der Bohrung 18 ein und schrägt die umlaufende Kante 54 umlaufend an, wie bei 60 in Fig. 7 zu erkennen. Insbesondere werden auch hier seitliche gegenüberliegende Flächenabschnitte 38, 40 geformt, welche die Kantenabschnitte der umlaufenden Kante 52 in diesem Bereich anschrägen oder anfasen, um die Spannungsspitzen in diesem Bereich des Nagelabschnitts herabzusetzen. Mit anderen Worten wird ein Verriegelungsnagel, insbesondere für Frakturen des proximalen Femurs beschrieben, bei dem im proximalen Abschnitt des Nagels eine vorzugsweise schräg zur Achse des proximalen Nagelabschnitts verlaufende Bohrung vorgesehen ist zur Aufnahme einer Schraube oder eines Stiftes, insbesondere eines Schenkelhalsstiftes, wobei die Kante des Eintrittsendes 54 eine umlaufende Fase 60 bzw. Schrägfläche aufweist. Dessen Form und Verlauf ist dadurch gebildet, dass mit Hilfe eines kreisförmigen Fräswerkzeugs 56, das sich um eine Achse senkrecht zur Achse des proximalen Abschnitts 12 dreht und das einen Aussendurchmesser hat, der grösser ist als der Durchmesser der Bohrung 18 annähernd mittig gegen das Eintrittsende gegen die Kante 52 des Eintrittsendes 54 annähernd senkrecht zur Achse des proximalen Nagelabschnitts 50 bewegt wird, bis die umlaufende Fase 60 erzeugt ist.

Zur Verdeutlichung sei angemerkt, dass die umlaufende Kante des Eintrittsbereichs eine umlaufende Fase bzw. Schrägfläche aufweist, deren Form und Verlauf dadurch gebildet ist, dass mit Hilfe eines kreisförmigen Fräswerkzeugs, das um seine Achse gedreht wird, die senkrecht auf der Achse des proximalen Nagelabschnitts steht und dessen Durchmesser grösser ist als der Durchmesser der Bohrung, gegen die Kante des Eintrittsendes bewegt wird, bis die umlaufende Fase erzeugt ist. Auf diese Weise wird ebenfalls eine wirksame Spannungsentlastung im Eintrittsende der Bohrung erzielt, ohne dass es zu einer ungünstigen Materialschwächung kommt oder die Auflagefläche des Schenkelhalsstiftes oder eines anderen Stiftes in der Bohrung verringert wird. Der Fertigungsschritt, der hierfür erforderlich ist, ist auf einfache Weise mit einfachen Mitteln möglich.

## Patentansprüche

1. Verriegelungsnagel, insbesondere für Frakturen des proximalen Femurs, bei dem im proximalen Abschnitt des Nagels eine vorzugsweise zur Achse des proximalen Abschnitts verlaufende Bohrung vorgesehen ist zur Aufnahme einer Schraube oder eines Stifts, insbesondere eines Schenkelhalsstiftes, **dadurch gekennzeichnet, daß** die auf gegenüberliegenden Seiten liegenden seitlichen Kantenabschnitte am Eintrittsende (30) oder auch Austrittsende der Bohrung (18) abgeflacht sind und gerundet und stetig in benachbarte Kantentabschnitte zur proximalen und distalen Seite des Eintritts- und/oder Austrittsendes hin übergehen.

2. Verriegelungsnagel nach Anspruch 1, **dadurch gekennzeichnet, daß** die durch die abgeflachten Kantenabschnitte gebildeten Flächenabschnitte (38, 40) in Längsrichtung des Nagelabschnitts im Endbereich im wesentlichen konkav verlaufen.

3. Verriegelungsnagel nach Anspruch 1 oder 2, **dadurch gekennzeichnet, daß** am Eintrittsende (30) und/oder Austrittsende der Bohrung (18) zur Bildung der abgeflachten Kantenabschnitt eine Kerbe geformt ist.

4. Verriegelungsnagel nach Anspruch 3, **dadurch gekennzeichnet, daß** die Kerbe derart geformt ist, daß die Gleitfläche des Stiftes in der Bohrung (18) im wesentlichen auf der ganzen Länge der ursprünglichen Bohrung (18) erhalten bleibt.

5. Verriegelungsnagel nach Anspruch 3 oder 4, **dadurch gekennzeichnet, daß** die umlaufende äußere im Querschnitt gerundete Kante (34) der Kerbe eine annähernd reckteckige oder quadratische Kontur hat.

6. Verriegelungsnagel nach einem der Ansprüche 2 bis 5, **dadurch gekennzeichnet, daß** die annähernd achsparallel verlaufenden seitlichen Flächenabschnitte (38, 40) im mittleren Bereich - in Längsrichtung des proximalen Nagelabschnitts gesehen - annähernd in einer gemeinsamen Ebene liegen, zu der die Längsachse des proximalen Nagelabschnitts (12) annähernd parallel verläuft.

7. Verriegelungsnagel nach einem der Ansprüche 3 bis 6, **dadurch gekennzeichnet, daß** die auf gegenüberliegenden Seiten des Stiftes liegenden seitlichen Flächenabschnitte (38, 40) der Kerbe zum proximalen Ende des Verriegelungsnagels hin gerundet in einem Querschnitt konkaven Flächenabschnitt (44) übergehen, dessen radial äußerer Kantenabschnitt den proximalen äußeren Kantenabschnitt der Kerbe bildet.

8. Verriegelungsnagel nach einem der Ansprüche 3 bis 7, **dadurch gekennzeichnet, daß** die auf gegenüberliegenden Seiten des Stiftes liegenden seitlichen Flächenabschnitte (38, 40) der Kerbe zum distalen Ende des Nagels hin gerundet in einen sich quer zur Nagelachse erstreckenden konkaven Flächenabschnitt übergehen, der annähernd parallel zur Bohrungsachse nach außen über eine gerundete Kante in die Außenkontur des Nagels (10) übergeht.

## Claims

1. Interlocking nail, in particular for fractures of the proximal femur, in which proximal portion of the nail there is a bore preferably extending to the axis of the proximal portion for receiving a screw or a pin, in particular a femoral neck pin, **characterised in that** the lateral edge portions lying on opposite sides are flattened at the inlet end (30) or also at the outlet end of the bore (18) and rounded and continuously pass over into adjacent edge portions toward the proximal and distal side of the inlet and/or outlet end.

2. Interlocking nail according to claim 1, **characterised in that** the surface portions formed by the flattened edge portions (38, 40) substantially extend concavely in the longitudinal direction of the nail portion in the end region.

3. Interlocking nail according to claim 1 or 2, **characterised in that** a notch is formed at the inlet end (30) and/or outlet end of the bore (18) to form the flattened edge portion.

4. Interlocking nail according to claim 3, **characterised in that** the notch is shaped so that the sliding surface of the pin in the bore (18) is substantially maintained over the entire length of the original bore (18).

5. Interlocking nail according to claim 3 or 4, **characterised in that** the circumferential outer edge (34) of the notch which has a rounded cross section has an approximately rectangular or square contour.

6. Interlocking nail according to any one of claims 2 to 5, **characterised in that** in the middle region, the surface portions (38, 40) extending approximately axially parallely-viewed in the longitudinal direction of the proximal nail portion - lie approximately in a common plane in relation to which the longitudinal axis of the proximal nail portion (12) extends approximately parallely.

7. Interlocking nail according to any one of claims 3 to 6, **characterised in that** toward the proximal end of the interlocking nail, the lateral surface portions (38, 40) of the notch lying on opposing sides of the pin are rounded and pass over into a surface portion (44) with a concave cross section, the radially outer edge portion of which forms the proximal outer edge of the notch.

8. Interlocking nail according to any one of claims 3 to 7, **characterised in that** toward the distal end of the nail, the lateral surface portions (38, 40) of the notch lying on opposing sides of the pin are rounded and pass over into a concave surface portion extending transversely to the nail axis, which passes outwardly approximately parallely to the bore axis into the outer contour of the nail (10) via a rounded edge.

## Revendications

1. Clou de fixation, destiné notamment à des fractures du fémur proximal, la partie proximale dudit clou étant, de préférence, pourvue d'un orifice foré qui est orienté vers l'axe de ladite partie proximale et qui sert à accueillir une vis ou une broche, notamment une broche de col de fémur, **caractérisé en ce que**, à l'entrée (30) ou également à la sortie de l'orifice foré (18), les parties des bords situées sur les faces latérales opposées sont aplaties et arrondies et que la transition entre lesdites parties et d'autres parties situées sur les côtés proximal et distal des bords de l'entrée et/ou de la sortie se fait d'une manière continue.

2. Clou de fixation selon la revendication 1, **caractérisé en ce que** les surfaces (38, 40) constituées par lesdites parties aplaties des bords suivent, par rapport au sens longitudinal de ladite partie du clou, une courbure qui, dans la section terminale, est essentiellement concave.

3. Clou de fixation selon les revendications 1 ou 2, **caractérisé en ce qu'**une entaille est réalisée à l'entrée (30) et/ou à la sortie de l'orifice foré (18) afin de former lesdites parties aplaties des bords.

4. Clou de fixation selon la revendication 3, **caractérisé en ce que** ladite entaille est réalisée telle que la surface de glissement de ladite broche dans l'orifice foré (18) continue de s'étendre essentiellement sur toute la longueur que l'orifice foré (18) présente à l'origine.

5. Clou de fixation selon les revendications 3 ou 4, **caractérisé en ce que** le bord périphérique (34) à coupe transversale arrondie qui constitue la limite extérieur de l'entaille est sensiblement de forme rectangulaire ou carrée.

6. Clou de fixation selon l'une quelconque des revendications 2 à 5, **caractérisé en ce que** les parties centrales des surfaces latérales (38, 40), qui sont sensiblement parallèles à l'axe, se situent approximativement dans le même plan - vu dans le sens longitudinal de la partie proximale du clou - ledit plan étant sensiblement parallèle à l'axe longitudinal de ladite partie proximale (12) du clou.

7. Clou de fixation selon l'une quelconque des revendications 3 à 6, **caractérisé en ce que** les surfaces latérales (38, 40) de l'entaille, qui sont situées sur des côtés opposés de la broche, se poursuivent, en progressant vers l'extrémité proximale du clou de fixation, par une surface (44) à coupe transversale concave, la zone de transition entre lesdites surfaces étant de forme arrondie et la partie radiale extérieure du bord de cette dernière surface constituant la partie extérieure du bord de l'entaille.

8. Clou de fixation selon l'une quelconque des revendications 3 à 7, **caractérisé en ce que** les surfaces latérales (38, 40) de l'entaille, qui sont situées sur des côtés opposés de la broche, se poursuivent, en progressant vers l'extrémité distale du clou, par une surface qui s'étend transversalement à l'axe du clou, la zone de transition entre lesdites surfaces étant de forme arrondie et que cette dernière surface se poursuit, en progressant sensiblement en parallèle à l'axe de l'orifice foré vers l'extérieur, par le contour extérieur du clou (10), cette dernière zone de transition étant constituée par un bord arrondi.
